# EUROPEAN PATENT SPECIFICATION

(11) **EP 4 032 558 B1**
(45) Date of publication and mention of the grant of the patent: **06.11.2024**
(21) Application number: 20866363.3
(22) Date of filing: 10.09.2020
(51) Int. Cl.: A61L 15/32, A61K 9/00, A61K 45/00, A61K 47/42, A61L 15/44, A61L 15/60, A61L 24/00, A61L 24/10, A61P 31/04, A61P 35/00, A61P 43/00, A61L 31/16, A61L 31/04

(54) **BIOLOGICAL TISSUE ADHESIVE SHEET, BIOLOGICAL TISSUE REINFORCING MATERIAL KIT, AND METHOD FOR MANUFACTURING BIOLOGICAL TISSUE ADHESIVE SHEET**
KLEBEFOLIE FÜR BIOLOGISCHES GEWEBE, KIT FÜR BIOLOGISCHES GEWEBEVERSTÄRKUNGSMATERIAL UND VERFAHREN ZUM HERSTELLEN EINER KLEBEFOLIE FÜR BIOLOGISCHES GEWEBE
FEUILLE ADHÉSIVE DE TISSU BIOLOGIQUE, KIT DE MATÉRIAU DE RENFORCEMENT DE TISSU BIOLOGIQUE ET PROCÉDÉ DE FABRICATION DE FEUILLE ADHÉSIVE DE TISSU BIOLOGIQUE

(30) Priority: 18.09.2019 JP 2019168903; 26.05.2020 JP 2020091006
(43) Date of publication of application: 27.07.2022
(73) Proprietor: National Institute for Materials Science, Tsukuba-shi, Ibaraki 305-0047 (JP)
(72) Inventor: TAGUCHI, Tetsushi, Tsukuba-shi, Ibaraki 305-0047 (JP)
(74) Representative: Dehns
(86) International application number: PCT/JP2020/034212
(87) International publication number: WO 2021/054232

(56) References cited:
- WO-A1-2014/112208
- WO-A1-2017/126390
- WO-A1-2020/071429
- JP-A- 2000 037 450
- JP-A- 2000 157 622
- JP-A- 2003 301 362
- JP-A- 2004 321 484
- JP-A- 2014 100 420
- YAMAOKA, MASATOSHI ET AL.: "Novel Alaska Pollock Gelatin Sealant Shows High Adhesive Quality and Conformability", ANN. THORAC. SURG., vol. 107, June 2019 (2019-06-01), pages 1656 - 1662, XP085694779, ISSN: 0003-4975, DOI: 10.1016/j.athoracsur.2018.11.074
- HIROAKI ICHIMARU, YOSUKE MIZUNO, CHEN XI, AKIHIRO NISHIGUCHI, TETSUSHI TAGUCHI: "P67A Development of a hydrophobically modified Alaska pollock-derived gelatin sheet possessing sealing effect for pulmonary air leaks", LECTURE ABSTRACTS OF THE 58TH SYMPOSIUM OF THE ADHESION SOCIETY OF JAPAN; JUNE 18-19, 2020, vol. 58, 18 June 2020 (2020-06-18) - 19 June 2020 (2020-06-19), JP, pages P67A, XP009534700

## Description

### Technical Field

The present invention relates to a biological tissue adhesive sheet, a biological tissue reinforcement material kit, and a method for producing a biological tissue adhesive sheet.

### Background Art

In medical fields such as thoracic surgery, gastroenterological surgery, and gastroenterological medicine, sheet-shaped medical devices (medical sheets) are used for the purpose of reinforcing sutures after surgery, preventing air leakage, and the like.

Patent Literature 1 describes, as such a medical sheet, "A suture prosthetic material comprising, in at least a part thereof, a nonwoven fabric that is made of polyglycolic acid and is produced by melt blowing".

### Citation List

### Patent Literature

Patent Literature 1: JP 2000-157622 A

### Summary of Invention

### Technical Problem

The present inventor and his colleagues have found that it may be difficult to have, with use of the suture prosthetic material described in Patent Literature 1, sufficient tissue adhesiveness in a wet environment at a biological tissue surface.

Therefore, in view of the above-mentioned circumstances, an object of the present invention is to provide a biological tissue adhesive sheet having excellent tissue adhesiveness.

Another object of the present invention is to provide a biological tissue reinforcement material kit and a method for producing a biological tissue adhesive sheet.

### Solution to Problem

As a result of intensive studies focusing on gelatins having excellent biocompatibility, the present inventor has found that the above-mentioned objects can be achieved by the following configuration.

The present invention provides a biological tissue adhesive sheet including a nonwoven fabric made of fibers containing a crosslinked gelatin derivative, the gelatin derivative being represented by Formula 1 shown below:

Formula 1: Gltn-NH-L-CHR¹R²

wherein Gltn represents a gelatin residue, L represents a single bond or a divalent linking group, R¹ and R² are each independently a hydrocarbon group having 1 to 20 carbon atoms or a hydrogen atom, and at least one selected from the group consisting of R¹ and R² is the hydrocarbon group, and
wherein the biological tissue adhesive sheet has a thickness in a range of 100 µm or more and 500 µm or less.
The hydrocarbon group in Formula 1 may be at least one selected from the group consisting of a chain hydrocarbon group having 1 to 20 carbon atoms, an alicyclic hydrocarbon group having 3 to 20 carbon atoms, an aromatic hydrocarbon group having 6 to 14 carbon atoms, and a group that is a combination of the foregoing groups and has 2 to 20 carbon atoms.

The hydrocarbon group may be an alkyl group having 1 to 20 carbon atoms.

The alkyl group may have 4 to 18 carbon atoms.

The biological tissue adhesive sheet may contain an agent fixed to the nonwoven fabric.

The agent may be an anticancer agent.

The agent may be an antimicrobial agent.

The agent may be a growth factor.

The gelatin derivative may be a derivative of a cold-water fish gelatin.

The cold-water fish gelatin may be at least one selected from the group consisting of cod gelatin, sea bream gelatin, salmon gelatin, and a genetically-engineered gelatin derived from cod, sea bream, or salmon.

The present invention also provides a biological tissue reinforcement material kit including the biological tissue adhesive sheet as described herein, and an instruction describing how to use the biological tissue adhesive sheet and/or precautions for use.

The present invention also provides a method for producing the biological tissue adhesive sheet as described herein, the method including:
preparing a composition containing the gelatin derivative and a solvent;
electrospinning the composition to prepare a nonwoven fabric made of fibers containing the gelatin derivative; and
applying energy to the nonwoven fabric to produce a biological tissue adhesive sheet having a thickness in a range of 100 µm or more and 500 µm or less.

The energy may be applied by heating the nonwoven fabric.

The method may further include irradiating the biological tissue adhesive sheet with an ultraviolet ray to hydrophilize surfaces of the fibers containing the gelatin derivative.

The biological tissue adhesive sheet may be irradiated with the ultraviolet ray for 10 minutes to 90 minutes.

### Advantageous Effects of Invention

According to the present invention, it is possible to provide a biological tissue adhesive sheet having excellent tissue adhesiveness. Moreover, in a predetermined burst strength test, the biological tissue adhesive sheet of the present invention exhibits a higher burst strength than a conventional nonwoven fabric made of a biodegradable absorbent material does.

Further, according to the present invention, it is also possible to provide a biological tissue reinforcement material kit and a method for producing a biological tissue adhesive sheet.

Since the biological tissue adhesive sheet of the present invention has a configuration including a nonwoven fabric made of fibers containing a crosslinked gelatin derivative, when the biological tissue adhesive sheet contains an agent (for example, an anticancer agent) fixed to the nonwoven fabric, the sheet can be used not only as a tissue reinforcement material but also as an agent eluting sheet.

### Brief Description of Drawings

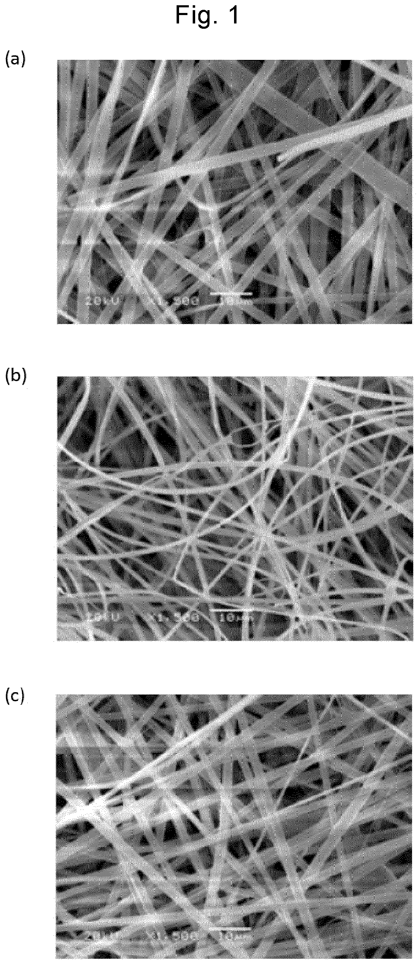

Fig. 1 shows SEM images of gelatin derivative fiber sheets of Examples 1 to 3; Fig. 1(a) shows a gelatin derivative fiber sheet of Example 1 (20C10-ApGltn), Fig. 1(b) shows a gelatin derivative fiber sheet of Example 2 (13C10-ApGltn), and Fig. 1(c) shows a gelatin derivative fiber sheet of Example 3 (37C10-ApGltn).

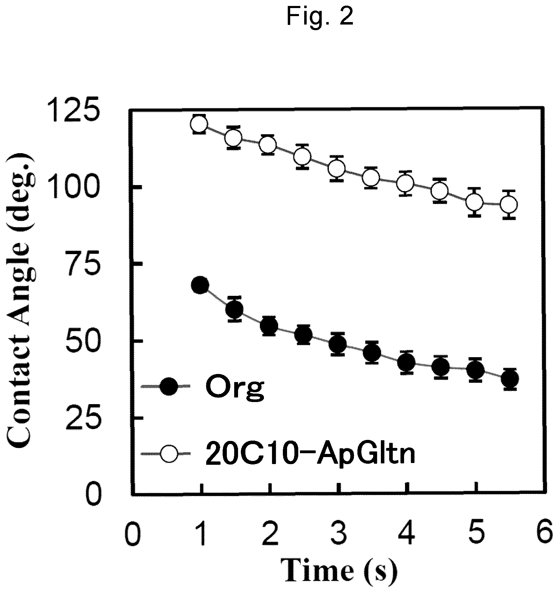

Fig. 2 is a graph showing a temporal change in contact angle with surfaces of the gelatin derivative fiber sheet of Example 1 and a gelatin fiber sheet of Reference Example 1. Open circles: Example 1 (20C10-ApGltn), filled circles: Reference Example 1 (Org)

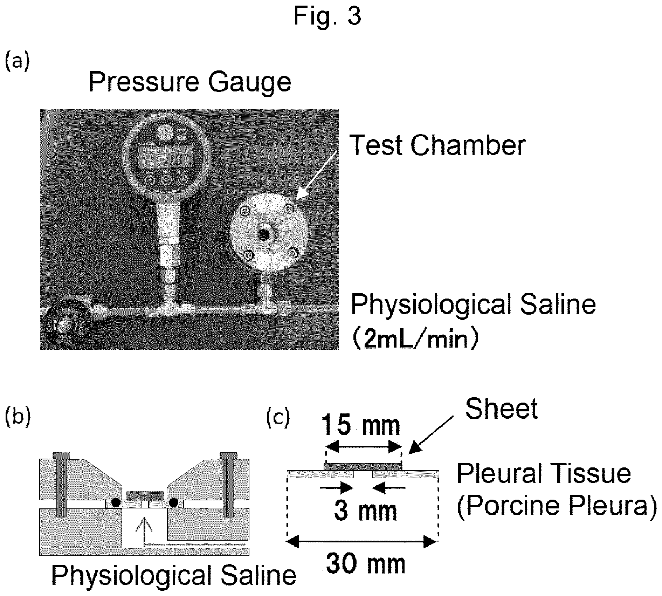

Fig. 3 shows an outline of a burst strength test on a porcine pleura in the section of Examples; Fig. 3(a) is a photograph showing a configuration of an apparatus used in the test, Fig. 3(b) is a schematic view showing a cross section of a test chamber, and Fig. 3(c) is a schematic view showing a configuration of an adherend (porcine pleura) and a gelatin derivative fiber sheet.

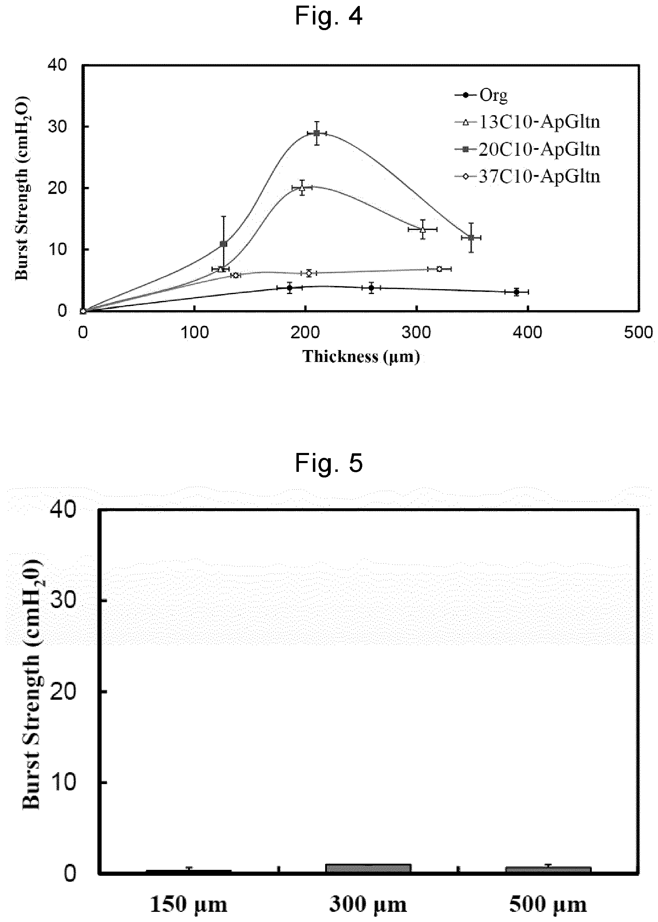

Fig. 4 is a graph showing a relation between the thickness (µm) and the burst strength (cmH₂O) of the gelatin derivative fiber sheets of Examples 1 to 3 and the gelatin fiber sheets of Reference Example 1 that were prepared with thermal crosslinking for 3 hours. Squares: Example 1 (20C10-ApGltn), open triangles: Example 2 (13C10-ApGltn), open circles: Example 3 (37C10-ApGltn), filled circles: Reference Example 1 (Org)
Fig. 5 is a graph showing a relation between the thickness (µm) and the burst strength (cmH₂O) of nonwoven fabrics of a comparative example that are made of polyglycolic acid.

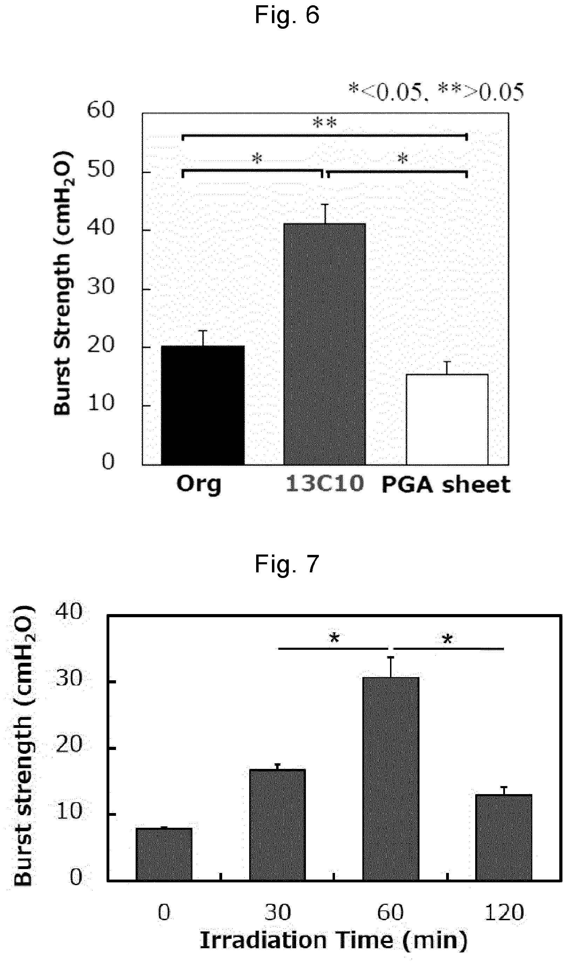

Fig. 6 is a graph showing results of a burst strength test on lungs isolated from a rat about the gelatin derivative fiber sheet (13C10) and the gelatin fiber sheet (Org) that were prepared with thermal crosslinking for 5 hours, and the nonwoven fabric made of polyglycolic acid (that is, a PGA sheet).
Fig. 7 is a graph showing a relation between the ultraviolet irradiation time (min) and the burst strength (cmH₂O) of gelatin derivative fiber sheets (8C10).

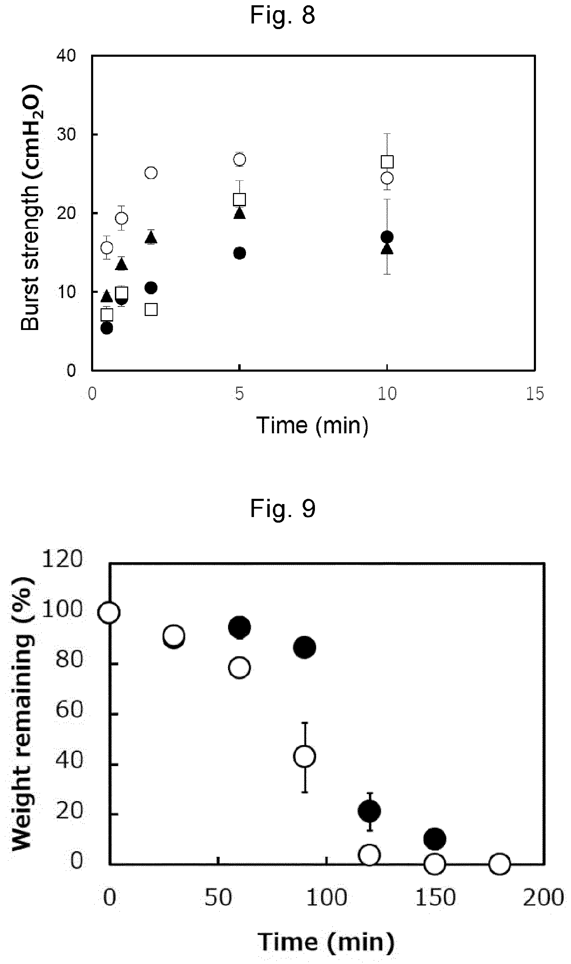

Fig. 8 is a graph showing a relation between the standing time (min) and the burst strength (cmH₂O) of a gelatin derivative fiber sheet (UV-8C10-AdFS1) and a gelatin fiber sheet (UV-Org-AdFS1) that were prepared with thermal crosslinking for 1 hour and ultraviolet irradiation for 60 minutes, and a gelatin derivative fiber sheet (8C10-AdFS1) and a gelatin fiber sheet (Org-AdFS1) that were not irradiated with ultraviolet rays in a burst strength test on a porcine pleura. Open circles: UV-8C10-AdFS1, open squares: 8C10-AdFS1, filled triangles: UV-Org-AdFS1, filled circles: Org-AdFS1
Fig. 9 is a graph showing results of a degradability test using a collagenase on a gelatin derivative fiber sheet (UV-8C10-AdFS) and a gelatin fiber sheet (UV-Org-AdFS) that were prepared with ultraviolet irradiation for 60 minutes. Open circles: UV-8C10-AdFS, filled circles: UV-Org-AdFS

### Description of Embodiments

Hereinafter, embodiments of the present invention will be described.

### [Biological Tissue Adhesive Sheet]

The biological tissue adhesive sheet according to an embodiment of the present invention includes a nonwoven fabric made of fibers containing a crosslinked gelatin derivative.

Hereinafter, the biological tissue adhesive sheet according to the present embodiment, which includes a nonwoven fabric made of fibers containing a crosslinked gelatin derivative, is sometimes referred to as a "gelatin derivative fiber sheet".

### <Gelatin Derivative>

In the biological tissue adhesive sheet of the present embodiment, the gelatin derivative is represented by Formula 1 shown below:

Formula 1: Gltn-NH-L-CHR¹R²

wherein Gltn represents a gelatin residue, L represents a single bond or a divalent linking group, R¹ and R² are each independently a hydrocarbon group having 1 to 20 carbon atoms or a hydrogen atom, and at least one selected from the group consisting of R¹ and R² is the hydrocarbon group.

The divalent linking group as L is not particularly limited, and examples thereof include -C(O)-, -C(O)O-, -OC(O)-, -O-, -S-, -NR- (wherein R represents a hydrogen atom or a monovalent organic group), an alkylene group, an alkenylene group (preferably having 2 to 10 carbon atoms), and combinations thereof. Among them, -C(O)- is preferred.

In Formula 1, N is not particularly limited, but is preferably derived from an ε-amino group of lysine (Lys) in the gelatin. Examples of a method of bonding *-CHR¹R² (* represents a bonding position) to an amino group of lysine with a linking group interposed therebetween or without a linking group (in other words, directly) include, as described later, a method using a so-called reductive amination reaction (a method using an aldehyde or a ketone) and a method using a Schotten-Baumann reaction (a method using an acid chloride) or the like.

In another form, N may be a group obtained by reacting, using a carbodiimide compound or the like, a compound having an amino group with a carboxy group of an amino acid in the gelatin.

Note that the structure -NH- in Formula 1 can be detected, for example, on the basis of a band around 3300 cm⁻¹ in FT-IR (Fourier transform infrared spectroscopy) spectra.

In Formula 1, one of R¹ and R² is preferably a hydrogen atom.

The hydrocarbon group having 1 to 20 carbon atoms is not particularly limited, and examples thereof include a chain hydrocarbon group having 1 to 20 carbon atoms, an alicyclic hydrocarbon group having 3 to 20 carbon atoms, an aromatic hydrocarbon group having 6 to 14 carbon atoms, and a group that is a combination of the foregoing groups and has 2 to 20 carbon atoms.

Examples of the chain hydrocarbon group having 1 to 20 carbon atoms include a methyl group, an ethyl group, a n-propyl group, an i-propyl group, a n-butyl group, a 2-methylpropyl group, a 1-methylpropyl group, and a t-butyl group.

From the viewpoint of obtaining a biological tissue adhesive sheet having an even better effect of the present invention, in particular, the number of carbon atoms of the chain hydrocarbon group is preferably 2 or more, more preferably 3 or more, still more preferably 6 or more, particularly preferably 7 or more, and most preferably 8 or more, and is preferably 19 or less, more preferably 18 or less, still more preferably 17 or less, particularly preferably 16 or less, even more preferably 15 or less, and most preferably 14 or less.

Examples of the alicyclic hydrocarbon group having 3 to 20 carbon atoms include a cyclopropyl group, a cyclopentyl group, a cyclohexyl group, an adamantyl group, and a norbornyl group.

The aromatic hydrocarbon group having 6 to 14 carbon atoms is not particularly limited, and examples thereof include a phenyl group, a tolyl group, and a naphthyl group.

The group that is a combination of the foregoing groups is not particularly limited, and examples thereof include aralkyl groups having 6 to 12 carbon atoms, such as a benzyl group, a phenethyl group, a naphthylmethyl group, and a naphthylethyl group.

From the viewpoint of obtaining a biological tissue adhesive sheet having an even better effect of the present invention, in particular, the hydrocarbon group having 1 to 20 carbon atoms in Formula 1 is preferably an alkyl group having 1 to 20 carbon atoms.

The number of carbon atoms of the alkyl group can be appropriately selected within the above-mentioned range according to the intended use, application site, and the like of the biological tissue adhesive sheet. More specifically, when a certain level of water resistance is required as characteristics of the biological tissue adhesive sheet, the number of carbon atoms of the alkyl group is preferably 4 or more, more preferably 6 or more, and still more preferably 8 or more. Meanwhile, from the viewpoint of reducing self-aggregation in the biological tissue adhesive sheet, the number of carbon atoms of the alkyl group is preferably 18 or less, more preferably 16 or less, and still more preferably 14 or less.

From the viewpoint of obtaining a biological tissue adhesive sheet having an even better effect of the present invention, the gelatin derivative represented by Formula 1 is preferably at least one gelatin derivative selected from the group consisting of Formulae 2 and 3 shown below, and is more preferably the gelatin derivative represented by Formula 2.

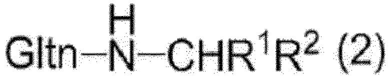

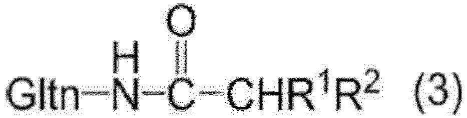

In Formulae 2 and 3, the meaning of each symbol is the same as that in Formula 1 described above, and the preferred form of the symbol is also the same.

In the gelatin derivatives represented by Formulae 2 and 3, *-CHR¹R² (* represents a bonding position, and hereinafter, the group represented by *-CHR¹R² is also referred to as a "hydrophobic group") is typically bonded to an amino group of lysine.

In the gelatin derivatives represented by Formulae 2 and 3, the amount of introduced hydrophobic groups is not particularly limited, but the content molar ratio of the amount of imino groups (-NH-) to which the hydrophobic group is bonded to the amount of amino groups in the raw material gelatin, that is, imino group/amino group is preferably 0.08 to 0.8, more preferably 0.08 to 0.7, still more preferably 0.08 to 0.6, and particularly preferably 0.08 to 0.5.

As used herein, the term "content molar ratio of imino group/amino group" (in other words, derivatization rate) means a numerical value that is obtained by quantifying the amount of amino groups in the raw material gelatin and the amount of amino groups in the gelatin derivative by a 2,4,6-trinitrobenzenesulfonic acid method (TNBS method).

The raw material gelatin usable for the preparation of the gelatin derivative is not particularly limited, and may be any gelatin obtained from natural origin or by synthesis, fermentation, genetic modification, or the like. More specifically, the raw material gelatin may be derived from a mammal or may be derived from a fish. From the viewpoint of obtaining a biological tissue adhesive sheet having an even better effect of the present invention, the gelatin derivative is preferably a derivative of a cold-water fish gelatin.

As used herein, the term "cold-water fish gelatin" refers to a gelatin derived from a cold-water fish.

It is known that habitat of cold-water fishes has a low water temperature (about 10°C to 15°C as a standard). Examples of the cold-water fish include, but are not limited to, cod, sea bream, and salmon. Here, the term "cod" is a generic name of fishes belonging to Gadinae, and examples thereof include Pacific cod, Saffron cod, and Alaska pollock. The term "sea bream" is a generic name of fishes belonging to Sparidae, and examples thereof include red sea bream, black sea bream, and yellowback sea bream. The term "salmon" is a generic name of fishes belonging to Salmonidae, and examples thereof include chum salmon, Atlantic salmon, and sockeye salmon.

The cold-water fish may be a natural fish or a genetically-engineered fish.

In a preferred embodiment, the cold-water fish gelatin is a gelatin derived from cod (cod gelatin). A gelatin derived from Alaska pollock is more preferred.

The cold-water fish gelatin may be produced from raw material collagen (collagen of a cold-water fish) that is pretreated with an acid (inorganic acid such as hydrochloric acid or sulfuric acid) or an alkali (lime). The pretreatment on the raw material collagen affects the hydrolysis of acid amide (that is, deamidation of amino acid side chains) in the collagen that occurs in the process of producing a gelatin. Therefore, the isoionic point of the gelatin varies depending on the presence or absence and conditions of the pretreatment. Specifically, the former acid-treated gelatin has an isoionic point of about pH 8 to 9, and the latter alkali-treated gelatin has an isoionic point of about pH 5.

The cold-water fish gelatin has an amino acid sequence similar to that of a gelatin derived from a mammal, is easily degraded by an enzyme, and has high biocompatibility. At the same time, the cold-water fish gelatin has a lower freezing point than that of a gelatin derived from a mammal, and has normal temperature fluidity. This is because the number (content) of hydroxyproline residues in the collagen of cold-water fishes is smaller than that in the collagen of mammals, and thus the collagen of cold-water fishes has a lower denaturation temperature. In addition, among fishes that are heterothermic animals, the denaturation temperature of collagen varies depending on the environment in which fishes are grown. Therefore, a fiber sheet containing a cold-water fish gelatin has a feature that the sheet is easily hydrated and gelates in vivo.

Advantages achieved when the biological tissue adhesive sheet of the present embodiment is a cold-water fish gelatin derivative fiber sheet include physical properties of the cold-water fish gelatin as described above. Therefore, it is considered more effective to select the kind of the cold-water fish gelatin according to the intended use, application site, and the like of the biological tissue adhesive sheet.

The molecular weight of the raw material gelatin is not particularly limited, but is preferably in the range of 20,000 to 150,000, and more preferably in the range of 30,000 to 100,000. When the molecular weight is within the above-mentioned range, a biological tissue adhesive sheet having even better tissue adhesiveness is obtained, the sheet itself is excellent in strength determined by a predetermined burst strength test as described in the section of Examples described later (hereinafter, the strength is also referred to as "film strength"), and the sheet has good handleability. As used herein, the term "molecular weight of the raw material gelatin" means the weight average molecular weight (Mw) in terms of standard pullulan measured by gel permeation chromatography (GPC).

The raw material gelatin may be a gelatin reduced in endotoxin content by a conventionally known method.

### <Nonwoven Fabric>

In the biological tissue adhesive sheet of the present embodiment, the nonwoven fabric is made of fibers containing a crosslinked gelatin derivative (hereinafter, the fibers are also referred to as "gelatin derivative fibers").

As used herein, the term "crosslinked gelatin derivative" means a crosslinked product of a gelatin derivative. The crosslinked product of a gelatin derivative typically means a reaction product that is obtained by applying energy such as heat or light to the gelatin derivative and/or reacting the gelatin derivative with a crosslinking agent.

As used herein, the term "crosslinked gelatin derivative" or "crosslinked product of a gelatin derivative" means a gelatin derivative in a state of being crosslinked by an irreversible crosslinking reaction, that is, a crosslinked product of a gelatin derivative that is obtained by an irreversible crosslinking reaction, and does not encompass a gelatin derivative having a crosslinked structure (physical crosslinked structure) formed by an intermolecular and/or intramolecular interaction of the gelatin derivative.

The method of applying energy to the gelatin derivative to produce a crosslinked product of the gelatin derivative is not particularly limited, and examples thereof include a method of applying heat (that is, a method of applying thermal energy), and a method of applying an active ray or radiation (for example, an electron beam) (that is, a method of applying light energy). Among them, a method of applying thermal energy (in other words, heating) (that is, a thermal crosslinking method) is preferred from the viewpoint of more easily obtaining a crosslinked product of the gelatin derivative.

The method of thermally crosslinking the gelatin derivative is not particularly limited, and typical examples thereof include a method of heating the gelatin derivative in a reduced pressure environment at 100 to 200°C for 1 to 8 hours. In the above-mentioned method, for example, an amino group in the gelatin derivative and other reactive groups (for example, a carboxy group and a mercapto group) undergo a reaction to produce a crosslinked product.

The crosslinked product of a gelatin derivative may be obtained by reacting a gelatin derivative with a crosslinking agent. The crosslinking agent is not particularly limited, and examples thereof include genipin, a polybasic acid activated with N-hydroxysuccinimide or N-sulfoxysuccinimide, an aldehyde compound, an acid anhydride, dithiocarbonate, and diisothiocyanate.

Examples of the usable crosslinking agent also include the compounds described in paragraphs [0021] to [0024] of WO 2018/079538 A1, the content of which is incorporated herein.

The thickness of the present biological tissue adhesive sheet can be appropriately adjusted according to the intended use, application site, and the like. Specifically, for example, when the biological tissue adhesive sheet is applied to an organ such as a lung in respiratory surgery or gastroenterological surgery, the thickness is in the range of 100 µm or more and 500 µm or less. When the thickness is within the above-mentioned range, the biological tissue adhesive sheet has both excellent bulk strength and excellent flexibility.

When the thickness is 100 µm or more, the biological tissue adhesive sheet has better bulk strength. When the thickness is 500 µm or less, the biological tissue adhesive sheet has better flexibility.

In particular, from the viewpoint of obtaining a biological tissue adhesive sheet having even better bulk strength, the thickness is preferably 150 µm or more. From the viewpoint of obtaining a biological tissue adhesive sheet having even better flexibility, the thickness is preferably 400 µm or less, more preferably 300 µm or less, and still more preferably 250 µm or less.

### <Agent>

In one aspect, the biological tissue adhesive sheet of the present embodiment preferably contains an agent fixed to the nonwoven fabric. The term "fix" also encompasses to "encapsulate" and "adsorb" the agent.

The agent is selected according to the intended use, application site, and the like of the biological tissue adhesive sheet. Examples of the agent include, but are not limited to, an anticancer agent, an antimicrobial agent, and a growth factor. One kind of agent may be used alone, or two or more kinds thereof may be used in combination.

Examples of the anticancer agent include, but are not limited to, cisplatin, carboplatin, nedaplatin, and oxaliplatin. Note that the above-mentioned anticancer agents are classified into platinum complexes (platinum compounds), but the anticancer agent usable in the present invention is not limited thereto, and examples thereof may include: alkylating agents (such as nitrogen mustards (e.g., cyclophosphamide) and nitrosoureas (e.g., nimustine)); antimetabolites (such as antifolates (e.g., pemetrexed), pyrimidine metabolism inhibitors, purine metabolism inhibitors, and nucleotide analogs); topoisomerase inhibitors; microtubule inhibitors; antitumor antibiotics (such as doxorubicin); and molecular target agents.

Examples of the antimicrobial agent include antibacterial antibiotics and antifungal antibiotics. More specific examples include, but are not limited to, antimicrobial agents classified into penicillins (such as Penicillin G, ampicillin, and bacampicillin), cephems (such as first generation (e.g., cefazolin), second generation (e.g., cefotiam), third generation (e.g., cefdinir), and fourth generation (e.g., cefepime)), carbapenems, monobactams, and penems. In addition to the above-mentioned antimicrobial agents that are also comprehensively referred to as β-lactam antimicrobial agents, for example, aminoglycoside, lincomycin, chloramphenicol, macrolide, ketolide, polypeptide, glycopeptide, and tetracycline antimicrobial agents can also be used. Moreover, synthetic antimicrobial agents classified into quinolones, oxazolidinones, sulfa agents, and the like can also be used.

Examples of the growth factor include, but are not limited to, vascular endothelial growth factors (VEGFs), basic fibroblast growth factors (bFGFs), platelet-derived growth factors (PDGFs), hepatocyte growth factors (HGFs), and transforming growth factors-β (TGF-βs).

The agent may be subjected to a sustained release treatment by a conventionally known method as necessary. Owing to the sustained release treatment, when the agent is, for example, an anticancer agent, the effect of the anticancer agent on the target cancer cell can be further enhanced.

Here, as an application example of the biological tissue adhesive sheet of the present embodiment, treatment of malignant pleural mesothelioma will be described as an example.

Pleural mesothelioma causes, even in the case where it is possible to apply surgery (resection) by extrapleural pneumonectomy (EPP) or pleurectomy/decortication (P/D), postoperative recurrences in many cases, and is known to be one of malignant diseases having poor prognosis.

The biological tissue adhesive sheet of the present embodiment has excellent adhesiveness to surfaces of soft tissues including the pulmonary pleura in a wet environment, and contains, as the agent fixed to the nonwoven fabric, an anticancer agent such as cisplatin. Therefore, it is considered that the biological tissue adhesive sheet can release the anticancer agent sustainedly, and can kill cancer cells remaining after resection of a cancer lesion.

As described above, the biological tissue adhesive sheet of the present invention is capable of having excellent biocompatibility and tissue adhesiveness, and also having sustained agent releasability. Therefore, the biological tissue adhesive sheet can be applied not only to use for the purpose of reinforcing sutures after surgery, preventing air leakage, and the like as with conventional medical sheets, but also to use for the purpose of treating a biological tissue after surgery.

### [Method for Producing Biological Tissue Adhesive Sheet]

The method for producing a biological tissue adhesive sheet is not particularly limited, and typical examples thereof include a method of producing a nonwoven fabric by a conventionally known method such as electrospinning, melt blowing, or spunbonding using a gelatin derivative, and then crosslinking the gelatin derivative.

In particular, the following production method is preferred as the method for producing the present biological tissue adhesive sheet in that a biological tissue adhesive sheet having excellent uniformity is more easily obtained.

A method for producing a biological tissue adhesive sheet according to an embodiment of the present invention (hereinafter, the method is also referred to as "the present production method") includes:
preparing a composition containing a gelatin derivative and a solvent (hereinafter, the step is also referred to as a "composition preparation step");
electrospinning the composition to prepare a nonwoven fabric made of fibers containing the gelatin derivative (hereinafter, the step is also referred to as an "electrospinning step"); and
applying energy to the nonwoven fabric to produce a biological tissue adhesive sheet having a thickness in a range of 100 µm or more and 500 µm or less (hereinafter, the step is also referred to as an "energy application step").

Hereinafter, the above-mentioned steps will be described.

### (Composition Preparation Step)

The composition contains the gelatin derivative described above, and a solvent. The content of the gelatin derivative in the composition is not particularly limited, but from the viewpoint of obtaining a biological tissue adhesive sheet having an even better effect of the present invention, the content is generally preferably 5 to 50 mass/volume% (g/mL) with respect to the total volume-based amount (mL) of the solvent in the composition. The composition may contain one kind of gelatin derivative alone or two or more kinds thereof. When the composition contains two or more kinds of gelatin derivatives, it is preferred that the total content of the gelatin derivatives be within the above-mentioned numerical range.

The solvent contained in the composition is not particularly limited, and a solvent capable of dissolving and/or dispersing the gelatin derivative is preferred. Examples of the usable solvent include water, organic solvents, and mixtures thereof. Examples of the usable water include ultrapure water, deionized water, and distilled water. The organic solvent is not particularly limited, and alcohols having 1 to 3 carbon atoms, esters, and the like can be used. Ethanol is preferred.

The method for preparing the composition is not particularly limited, and a known method can be used. For example, the gelatin derivative may be added to the solvent and heated as necessary.

The heating temperature in this case is preferably a temperature at which thermal crosslinking of the gelatin derivative does not proceed. Specifically, the heating temperature is preferably 40 to 90°C.

The composition may contain components other than those described above. Examples of the components other than those described above include a crosslinking agent and an agent.

Depending on the intended use, application site, and the like of the biological tissue adhesive sheet, it is preferred that the composition do not substantially contain a crosslinking agent from the viewpoint that an unintended action by an unreacted crosslinking agent is less likely to occur.

A method for synthesizing the gelatin derivative is not particularly limited, and examples thereof include a synthesis method described below, which includes (1) preparation of a raw material gelatin solution, (2) derivatization, and (3) purification.

### (1) Preparation of raw material gelatin solution

A raw material gelatin in an amount of 5 to 50 mass/volume% with respect to a solvent (water and/or an organic solvent miscible with water) is heated at 40 to 90°C to be dissolved in the solvent to give a gelatin solution.

Examples of the usable water include ultrapure water, deionized water, and distilled water.

The organic solvent is not particularly limited, and alcohols having 1 to 3 carbon atoms, esters, and the like can be used. Ethanol is preferred.

### (2) Derivatization

To the gelatin solution obtained in the item (1), a derivatization reagent having a hydrocarbon group to be introduced is added, and the resulting mixture is stirred for a predetermined time to cause a reaction. Examples of the usable derivatization reagent include hydrocarbon group-containing aldehydes and ketones. Reagents in which an amino group (for example, a primary amino group) is further bonded to a hydrocarbon group can also be used.

When a hydrocarbon group-containing aldehyde, ketone, or the like is used as the derivatization reagent, the gelatin derivative of Formula 1 is obtained using so-called "reductive amination".

When a compound having a hydrocarbon group and an amino group bonded to the hydrocarbon group is used as the derivatization reagent, the gelatin derivative of Formula 1 is obtained by bonding the hydrocarbon group to a carboxy group of the gelatin via an imino group using a carbodiimide compound.

The hydrocarbon group-containing aldehyde, ketone, or the like is not particularly limited, and examples of the usable compound include hexanal, heptanal, octanal, nonanal, decanal, undecanal, dodecanal, tetradecanal, and decyl ethyl ketone. In this case, the reaction temperature is 30 to 80°C and the reaction time is 0.5 to 12 hours. Usually, a gelatin in which the hydrocarbon group is bonded to the amino group via a Schiff base (~N=CR¹R²) is obtained only by stirring. The amount of the aldehyde used is 1 to 4 times the stoichiometric amount corresponding to the desired derivatization rate. The amount of the aldehyde is more preferably 1 to 2 times.

Subsequently, the Schiff base is reduced to give the structure of Formula 1. Examples of the usable reducing agent include known reducing agents such as sodium cyanoborohydride (NaBHsCN), sodium triacetoxyborohydride (NaBH(OAc)₃), 2-picoline borane, and pyridine borane.

Among them, 2-picoline borane is preferred. Picoline borane is stable and can cause the reductive amination reaction of an aldehyde or a ketone in an organic solvent in one step (one pot). In addition, picoline borane can achieve a yield of 80 to 90%.

The amount of 2-picoline borane used is preferably 1 to 3 equivalents with respect to the equivalent of the derivatization reagent. The order of adding the reducing agent and the aldehyde or the like may be arbitrary, and either of them may be added first to the gelatin solution or both of them may be added simultaneously.

As another form of derivatization, a Schotten-Baumann reaction can also be used. The Schotten-Baumann reaction is a method of reacting a carboxylic acid chloride with an amine in the presence of a base to give an amide. Using the Schotten-Baumann reaction, typically, an amide bond derived from the ε-amino group of lysine is formed, and a predetermined hydrophobic group can be introduced into the derivative.

More specifically, it is preferred to add a base to the gelatin solution, dissolve a carboxylic acid chloride in an organic solvent, and mix them to advance a condensation reaction.

The base is not particularly limited, and water-soluble bases such as triethylamine and pyridine can be generally used.

### (3) Purification

A large excess of a poor solvent such as cold ethanol is added to the reaction solution obtained in the item (2), or the reaction solution is added to cold ethanol to precipitate a crude gelatin derivative. The precipitate is filtered off and then washed with ethanol or the like to give a gelatin derivative.

### (Electrospinning Step)

The electrospinning step is a step of electrospinning the composition to prepare a nonwoven fabric.

In the electrospinning, a high voltage is applied to the composition so that the composition may be charged to form fibers, and the fibers are deposited to form a nonwoven fabric. As for a method for charging the composition, it is preferred to connect an electrode connected to a high-voltage power supply device to the composition itself or a container, and typically apply a voltage of 1 to 100 kV, and it is more preferred to apply a voltage of 5 to 50 kV.

The kind of voltage may be either direct current or alternating current.

The temperature during electrospinning is not particularly limited, and may be appropriately adjusted according to the boiling point and volatility of the solvent contained in the composition. In one embodiment, the temperature is preferably 10 to 30°C.

Since the present production method includes this step, the nonwoven fabric is produced without heating the composition, in other words, without heating the gelatin derivative. Therefore, unintentional crosslinking of the gelatin derivative is reduced, and a biological tissue adhesive sheet having a more uniform structure (more uniform fiber diameter or the like) is easily obtained.

### (Energy Application Step)

The energy application step is a step of applying energy to the nonwoven fabric to produce a biological tissue adhesive sheet. Owing to the application of energy, at least a part of the gelatin derivative undergoes intermolecular and/or intramolecular crosslinking, and the resulting biological tissue adhesive sheet has even better bulk strength and even better water resistance.

The energy to be applied is not particularly limited, and may be light and/or heat. In particular, it is preferred that the energy be applied by heating the nonwoven fabric from the viewpoint of more easily obtaining the biological tissue adhesive sheet.

The heating method is not particularly limited, and typical examples thereof include a method of heating the nonwoven fabric in a reduced pressure environment at 100 to 200°C for 1 to 8 hours. More specifically, for example, when the raw material gelatin has a molecular weight (Mw) of about 100,000, the nonwoven fabric is heated for 1 to 6 hours in a reduced pressure environment at 140 to 160°C (for example, 150°C).

Furthermore, the present production method may include irradiating the biological tissue adhesive sheet obtained by the energy application step with ultraviolet rays to hydrophilize surfaces of the fibers containing the gelatin derivative (the step is also referred to as an "ultraviolet irradiation step").

Hereinafter, the ultraviolet irradiation step will be described.

### (Ultraviolet Irradiation Step)

The ultraviolet irradiation step is a step of further irradiating the fibers containing the crosslinked gelatin derivative (that is, gelatin derivative fibers) with ultraviolet rays to hydrophilize surfaces of the fibers. The surface treatment by ultraviolet irradiation reduces the contact angle between the fibers and a water droplet, and the fibers are more easily swollen in the presence of moisture. Meanwhile, when the fibers are brought into contact with a tissue in a dry state, the fibers after ultraviolet irradiation still have excellent adhesiveness.

The conditions for ultraviolet irradiation are not particularly limited, and the fibers are usually irradiated for 10 minutes to 90 minutes, more preferably for 30 minutes to 90 minutes, and still more preferably for 45 minutes to 75 minutes. When the ultraviolet irradiation time is within the above-mentioned range, the surfaces of the gelatin derivative fibers can be appropriately hydrophilized without impairing the production efficiency.

The ultraviolet irradiation time is preferably adjusted within the above-mentioned range in consideration of the structure of the gelatin derivative that constitutes the gelatin derivative fibers. For example, when the gelatin derivative is represented by Formula 1, 2, or 3, it is preferred to consider the number of carbon atoms of the hydrophobic group in the gelatin derivative, the amount of hydrophobic groups introduced into the gelatin derivative, and the like. More specifically, in the section of Examples described later, in the case where gelatin derivatives in which the total number of carbon atoms of the hydrophobic groups (*-CHR¹R²) was 10 and the percentage of the introduced hydrophobic groups was 8 mol% were used and the fibers were irradiated with ultraviolet rays for 10 minutes, 30 minutes, or 60 minutes under predetermined conditions, for all the ultraviolet irradiation times, the contact angle of a water droplet dropped on the surface of the gelatin derivative fiber sheet was smaller than that in the case of no irradiation. In particular, the degree of decrease in the contact angle between 10 minutes and 30 minutes was very large. On the other hand, when a raw material gelatin in which no hydrophobic group was introduced was used, the contact angle of a water droplet after 5 minutes of ultraviolet irradiation was about the same as the value after 30 minutes of ultraviolet irradiation to the above-mentioned gelatin derivatives. More specifically, the degree of hydrophilization of the surfaces of the gelatin derivative fibers can be adjusted by adjusting the ultraviolet irradiation time within the above-mentioned range in consideration of the steric bulkiness, positional relation (density), and the like of the hydrophobic groups introduced into the gelatin derivative.

The ultraviolet intensity is preferably 0.05 to 50 mW/cm², and more preferably 0.5 to 10 mW/cm². The integrated light quantity of ultraviolet rays is preferably 1 to 100 J/cm², and more preferably 5 to 100 J/cm².

The device for ultraviolet irradiation is not particularly limited, and a commercially available ultraviolet irradiation device may be used. Usually, it is sufficient to irradiate one surface (surface that comes into contact with a target tissue) of the biological tissue adhesive sheet with ultraviolet rays, but both surfaces of the sheet may be irradiated with ultraviolet rays. In this case, the surfaces to be irradiated (front and back surfaces of the sheet) are preferably switched at a certain time interval (for example, every 5 minutes, every 10 minutes, or every 15 minutes). In addition, since the fiber surface is hydrophilic after ultraviolet irradiation, it is preferred to store the sheet in a dry atmosphere such as in the presence of a dehumidifier.

### [Biological Tissue Reinforcement Material Kit]

The biological tissue reinforcement material kit according to an embodiment of the present invention includes the biological tissue adhesive sheet.

The kit of the present embodiment includes an instruction describing how to use the biological tissue adhesive sheet of the present invention, precautions for use, and the like.

As used herein, the term "instruction" encompasses a package insert, a package label, and a direction for use, and is not particularly limited. Examples of the instruction include packaging insertions, prescribing information, and leaflets. The instruction may be provided on a paper medium or may be provided in a form such as an electronic medium (for example, a website or an e-mail provided on the Internet).

Hereinafter, the present invention will be described in more detail with reference to examples. Materials, amounts used, proportions, contents of treatment, treatment procedures, and the like shown in the following examples can be appropriately changed without departing from the gist of the present invention. Therefore, the scope of the present invention should not be restrictively interpreted by the following examples.

### Examples

### <Example 1>

### [Preparation of Gelatin Derivative]

To a 10% aqueous ethanol solution, 30 g of a gelatin derived from Alaska pollock (Mw = 33,000, manufactured by Nitta Gelatin Inc., hereinafter also referred to as a "raw material gelatin") was added, and the resulting mixture was stirred on an oil bath set at 55°C to dissolve the gelatin.

Then, 1.5 equivalents of decanal (C₁₀H₂₀O) in a stoichiometric amount corresponding to a derivatization rate of 50 mol% with respect to the amino groups in the gelatin was dissolved in 15 mL of ethanol, and the resulting solution was added to the gelatin solution. The solution was stirred for 1 hour, and then a solution of about 1.5 equivalents of 2-picoline borane with respect to decanal in 15 mL of ethanol was added thereto, and the resulting mixture was stirred for 17 hours.

Thereafter, the reaction solution was added dropwise to cold ethanol in an amount of 10 times the volume of the reaction solution to reprecipitate the produced gelatin derivative, and the resulting precipitate was subjected to suction filtration. The precipitate was placed in cold ethanol in an amount of about 5 times the volume of the precipitate, washed with stirring for 1 hour, and then subjected to suction filtration. The washing was performed 3 times, and then vacuum drying was performed for 2 days to give a white gelatin derivative having decyl groups introduced therein (yield: about 80 (mass/mass)%).

The percentage of the introduced decyl groups (that is, the derivatization rate) in the obtained gelatin derivative was quantified by the TNBS method.

Specifically, 5 mg of the gelatin derivative was added to 5 mL of a 50% aqueous DMSO solution, and dissolved by warming in a water bath set at 60°C for 1 hour. Thereafter, the gelatin derivative solution was aliquoted by 100 µL in a 48-well plate, 100 µL of an aqueous solution of 0.1% triethylamine (TEA) and 50% DMSO was added thereto, and the mixture was stirred on a plate shaker for 1 minute. Then, 100 µL of an aqueous solution of 0.1% TNBS and DMSO was added to the mixture, and the mixture was stirred on the plate shaker for 1 minute and then left standing at 37°C for 2 hours. Subsequently, 50 µL of 6 N HCl was added to stop the reaction. Finally, the absorbance at 340 nm was measured, and the amino group amount of the gelatin derivative was quantified.

The amino group amount was similarly quantified for the raw material gelatin, and it was recognized from the obtained results that the derivatization rate was 20 mol%.

Furthermore, an absorption spectrum was measured by Fourier transform infrared spectroscopy (FT-IR), and the molecular structure of the gelatin derivative was analyzed. As a result, an absorption around 2925 to 2970 cm⁻¹ derived from -CH₂- stretching vibration and an absorption around 2875 cm⁻¹ derived from -CHs- stretching vibration of an alkyl terminal were observed.

As a result of measuring the ¹H-NMR spectrum, methylene protons derived from an alkylene group were observed around 1.26 ppm.

The gelatin derivative obtained as described above was designated as "20C10-ApGltn". In the following description, the designation "a"C"b"-ApGltn, in which "a" and "b" are numbers, means that "b" is the total number of carbon atoms of the hydrophobic groups (*-CHR¹R²) in Formula 1, and "a" mol% is the percentage of the introduced hydrophobic groups.

In the following description, the designation "aCb" without the designation of the portion "-ApGltn" has the same meaning as described above.

### [Preparation of Gelatin Derivative Fiber Sheet]

To 5 mL of a 60% aqueous ethanol solution, 1.5 g of the obtained gelatin derivative (20C10) was added, and dissolved on a water bath set at 55°C. Then, the obtained gelatin solution was transferred to a syringe having a volume of 5 mL and equipped with an 18 gauge needle, and a nonwoven fabric made of gelatin derivative fibers was prepared on a collector by electrospinning.

The obtained nonwoven fabric was heated (thermally crosslinked) in a reduced pressure environment at 150°C for 3 hours to produce a gelatin derivative fiber sheet.

As a result of observation with a scanning electron microscope (SEM), it was recognized that the gelatin derivative fibers had a fiber diameter of 2 to 5 µm, and that a fiber sheet having a uniform structure was obtained (Fig. 1(a)). The sheet had a thickness of about 200 µm.

### <Example 2>

A gelatin derivative (13C10-ApGltn) having a derivatization rate of 13 mol% was prepared by the same procedure as in Example 1 except that the amount of decanal added to the raw material gelatin solution was changed.

Using the gelatin derivative (13C10), a gelatin derivative fiber sheet was prepared by the same procedure as in Example 1.

### <Example 3>

A gelatin derivative (37C10-ApGltn) having a derivatization rate of 37 mol% was prepared by the same procedure as in Example 1 except that the amount of decanal added to the raw material gelatin solution was changed.

Using the gelatin derivative (37C10), a gelatin derivative fiber sheet was prepared by the same procedure as in Example 1.

### <Reference Example 1>

A gelatin fiber sheet was prepared by the same procedure as in Example 1 using a raw material gelatin (hereinafter also referred to as "Org").

Figs. 1(a) to 1(c) are SEM images of the gelatin derivative fiber sheets of Examples 1 to 3, respectively.

As shown in Figs. 1(a) to 1(c), in all of the sheets, the gelatin derivative fibers had a fiber diameter of 2 to 5 µm, and defects such as spherical lumps (beads) were not observed.

Also in the gelatin fiber sheet of Reference Example 1, the fibers had a comparable fiber diameter, and defects such as spherical lumps (beads) were not observed.

In addition, 1 µL of pure water was dropped on the surfaces of the gelatin derivative fiber sheet of Example 1 and the gelatin fiber sheet of Reference Example 1, and the contact angle of the water droplet was measured over time. As a result, as shown in Fig. 2, the sheet (Org) of Reference Example 1 absorbed the pure water immediately after the dropping, whereas for the sheet (20C10) of Example 1, the pure water absorption speed was lower than that for Org, and an increase in the contact angle was observed.

### [Burst Strength Test on Porcine Pleura]

The gelatin derivative fiber sheets of Examples 1 to 3 and the gelatin fiber sheet of Reference Example 1 obtained as described above were subjected to a burst strength test using a pleura separated from a porcine lung as an adherend.

Figs. 3(a) to 3(c) show an outline of the burst strength test on the porcine pleura. Fig. 3(a) is a photograph showing a configuration of an apparatus used in the test, Fig. 3(b) is a schematic view showing a cross section of a test chamber, and Fig. 3(c) is a schematic view showing a configuration of the adherend (porcine pleura) and the gelatin derivative fiber sheet.

According to ASTM F2392-04 (Standard Test Method for Burst Strength of Surgical Sealants), a hole having a diameter of 3 mm was made in the center of the pleural tissue formed to have a diameter of 30 mm, and the gelatin derivative fiber sheet formed to have a diameter of 15 mm was attached to the pleural tissue (Fig. 3(c)). The tissue and the sheet were left standing for 10 minutes, and then placed in the test chamber (Fig. 3(b)).

Physiological saline was flowed at a flow rate of 2 mL/min from the direction indicated by the arrows in Figs. 3(a) and 3(b), and the pressure when the gelatin derivative fiber sheet attached to the pleural tissue was broken was measured by a pressure gauge (Fig. 3(a)).

As for the gelatin derivative fiber sheets of Examples 1 to 3, a total of three kinds of sheets, i.e., a sheet having a thickness of 100 to 150 µm, a sheet having a thickness of about 200 µm, and a sheet having a thickness of 300 to 350 µm, all prepared by the above-mentioned procedure, were used in each of the examples. As for the gelatin fiber sheet of Reference Example 1, a total of three kinds of sheets, i.e., a sheet having a thickness of about 200 µm, a sheet having a thickness of about 250 µm, and a sheet having a thickness of about 400 µm, all prepared by the above-mentioned procedure, were used.

In addition, as a comparative example, nonwoven fabrics (NEOVEIL (registered trademark) Type NV-M-015G (thickness: 150 µm), Type NV-M-03G (thickness: 300 µm), and Type NV-M-05G (thickness: 500 µm) manufactured by GUNZE LIMITED) made of polyglycolic acid were subjected to the above-mentioned burst strength test.

The results are shown in Figs. 4 and 5.

Fig. 4 is a graph showing a relation between the thickness (µm) and the burst strength (cmH₂O) of the gelatin derivative fiber sheets of Examples 1 to 3 and the gelatin fiber sheets of Reference Example 1 that were prepared with thermal crosslinking for 3 hours.

Fig. 5 is a graph showing a relation between the thickness (µm) and the burst strength (cmH₂O) of the nonwoven fabrics of the comparative example that are made of polyglycolic acid.

As shown in Fig. 4, the gelatin derivative fiber sheets of Examples 1 to 3 exhibited a superior burst strength to that of the gelatin fiber sheets of Reference Example 1 (n = 3). In particular, in the case of the sheets having a thickness of about 200 µm, the sheet (13C10) of Example 2 had a burst strength of about 20 (cmH₂O), and the sheet (20C10) of Example 1 had a burst strength of about 30 (cmH₂O).

On the other hand, as shown in Fig. 5, as for the nonwoven fabrics of the comparative example, in all the cases where the thickness was 150 µm, 300 µm, or 500 µm, the value of burst strength was only about 1 to 2 (cmH₂O) (n = 3), and the burst strength was significantly lower than those of the sheets (Org) of Reference Example 1 as well as the sheets of Examples 1 to 3.

It should be noted here that for the gelatin derivative fiber sheets produced in the examples of the present invention, it was possible to measure the pressure when the sheet was broken without separation at the adhesion site between the pleural tissue and the sheet while flowing the physiological saline in the test chamber, whereas for the nonwoven fabrics of the comparative example, separation occurred at the adhesion site between the pleural tissue and the sheet by flowing the physiological saline (n = 5). More specifically, the burst strength shown for the nonwoven fabrics of the comparative example should be understood more accurately not as "a pressure value when the nonwoven fabric attached to the pleural tissue was broken" (that is, a value indicating the film strength as referred to herein), but as "a pressure value when the nonwoven fabric attached to the pleural tissue was separated from the adhesion site" (that is, the pressure value when the adhesiveness to the adherend (biological tissue) was lost).

The separation at the adhesion site between the pleural tissue and the sheet caused by flowing the physiological saline was also observed in the case of using the gelatin fiber sheets of Reference Example 1.

From these results, it was confirmed that the gelatin derivative fiber sheet of the present invention is superior in tissue adhesiveness and film strength to conventional medical sheets.

From the results shown in Fig. 4, it was found that the biological tissue adhesive sheets of Example 1 having a thickness of 150 µm or more have even better adhesive strength. It was also found that the biological tissue adhesive sheets of Example 1 having a thickness of 500 µm or less have even better bulk strength and even better flexibility. It was also found that the biological tissue adhesive sheets of Example 1 having a thickness of 400 µm or less have still better bulk strength and still better flexibility. In addition, it was found that the biological tissue adhesive sheets of Example 1 having a thickness of 300 µm or less have particularly excellent bulk strength and particularly excellent flexibility.

From the results shown in Fig. 4, it was found that the biological tissue adhesive sheets of Example 1 in which the number of carbon atoms of the hydrophobic groups is 4 or more have even better bulk strength. It was also found that the biological tissue adhesive sheets of Example 1 in which the number of carbon atoms of the hydrophobic groups is 18 or less have even better bulk strength.

### <Example 4>

### [Preparation of Gelatin Derivative Fiber Sheets under Different Thermal Crosslinking Conditions and Characteristic Evaluation of the Sheets]

Using the gelatin derivative (20C10) of Example 1, nonwoven fabrics made of gelatin derivative fibers were prepared by the same procedure as described above, and then the nonwoven fabrics were subjected to thermal crosslinking for 1 hour or 5 hours to give gelatin derivative fiber sheets (thickness: about 200 µm). The gelatin derivative fiber sheets were subjected to the burst strength test.

As a result, it was recognized that the gelatin derivative fiber sheets had a film strength equivalent to that of the gelatin derivative fiber sheet obtained with thermal crosslinking for 3 hours (n = 3).

Using the gelatin derivative (37C10) of Example 3 having a derivatization rate of 37 mol%, gelatin derivative fiber sheets (thickness: about 200 µm) were prepared with thermal crosslinking for 1 hour or 5 hours. The gelatin derivative fiber sheets were subjected to the burst strength test. As a result, it was recognized that the gelatin derivative fiber sheets had a film strength equivalent to that of the gelatin derivative fiber sheet obtained with thermal crosslinking for 3 hours (n = 3) similarly to the case of the gelatin derivative (20C10) of Example 1.

On the other hand, using the gelatin derivative (13C10) of Example 2 having a derivatization rate of 13 mol%, gelatin derivative fiber sheets (thickness: about 200 µm) were prepared with thermal crosslinking for 1 hour or 5 hours. The gelatin derivative fiber sheets were subjected to the burst strength test. As a result, it was recognized that the gelatin derivative fiber sheet prepared with thermal crosslinking for 5 hours showed a value of about 40 (cmH₂O), and the film strength was improved to about 2 times that of the gelatin derivative fiber sheet obtained with thermal crosslinking for 3 hours (n = 3). It is to be noted that such improvement in film strength was also observed in the case of the sheet having a thickness of 100 to 150 µm and the case of the sheet having a thickness of 300 to 350 µm, but the improvement was particularly remarkable in the case of the sheet having a thickness of about 200 µm. It was also recognized that the gelatin derivative fiber sheet obtained with thermal crosslinking for 1 hour had a film strength equivalent to that of the gelatin derivative fiber sheet obtained with thermal crosslinking for 3 hours (n = 3).

Using the raw material gelatin (Org) of Reference Example 1, gelatin fiber sheets (thickness: about 200 µm) were prepared with thermal crosslinking for 1 hour or 5 hours. The gelatin fiber sheets were subjected to the burst strength test. As a result, it was recognized that the gelatin fiber sheets had a film strength equivalent to that of the gelatin fiber sheet obtained with thermal crosslinking for 3 hours (n = 3).

The fiber sheets before being subjected to the burst strength test were observed with a scanning electron microscope (SEM). As a result, both the fiber sheets had a fiber diameter of 1 to 3 µm and had a uniform structure.

From the results of the burst strength test on Examples 1 to 3 and this example, it was suggested that the film strength of the gelatin derivative fiber sheet can be adjusted by the derivatization rate in the gelatin, the thickness of the gelatin derivative fiber sheet, and the thermal crosslinking conditions (thermal crosslinking time).

More specifically, as the thermal crosslinking time is increased, the degree of progress of the crosslinking reaction between the amino group and the carboxy group in the gelatin derivative increases, and the crosslinking density in the gelatin derivative fiber sheet increases. The crosslinking density in the gelatin derivative fiber sheet correlates also with the derivatization rate in the gelatin and the thickness of the gelatin derivative fiber sheet. It may also be effective to adjust the thermal crosslinking temperature for adjusting the crosslinking density in the gelatin derivative fiber sheet. The gelatin derivative fiber sheet produced so as to have an appropriate crosslinking density as described above hardly swells in a wet environment at the surface of a biological tissue, and penetrates into a target biological tissue and is self-assembled to exhibit excellent adhesiveness, and thus has excellent burst strength and sealing strength.

### [Evaluation of Physical Properties]

On the surfaces of the gelatin derivative fiber sheets (13C10, 20C10, and 37C10) and the gelatin fiber sheet (Org) prepared with thermal crosslinking for 5 hours in this example, 1 µL of pure water was dropped, and the contact angle of the water droplet after 5 seconds was measured. As a result, it was recognized that the contact angle tended to increase in the order of Org, 13C10, 20C10, and 37C10.

A test piece was cut out from each sheet, and the test piece was immersed in physiological saline for 60 minutes to evaluate the degree of swelling. As a result, the sheets Org, 13C10, and 37C10 had almost the same swelling ratio (about 10%), whereas the sheet 20C10 showed a higher swelling ratio (about 20%).

The fiber sheets molded into a dumbbell shape in accordance with JIS K 7127: 1999 were subjected to a tensile test. As a result, the sheet Org showed the highest Young's modulus value (about 1.7 KPa), and the sheets 13C10, 20C10, and 37C10 had a Young's modulus in the range of about 0.7 to 1.0 KPa.

As for the tensile strength, the sheet Org showed the highest value (about 0.11 MPa), the sheets 13C10 and 37C10 showed a value of about 0.08 MPa, and the sheet 20C10 showed a value of about 0.05 MPa.

These physical properties are considered to reflect the crosslinking density in the gelatin derivative fiber sheet described above or the structural change of the gelatin due to heat treatment. Therefore, it is presumed that these physical properties tend to vary depending on the derivatization rate in the gelatin, the thickness of the gelatin derivative fiber sheet, the thermal crosslinking conditions (thermal crosslinking time), and the like.

### [Burst Strength Test on Lungs Isolated from Rat]

The gelatin derivative fiber sheet (13C10) and the gelatin fiber sheet (Org) prepared with thermal crosslinking for 5 hours in this example and the nonwoven fabric (thickness: 150 µm) of the comparative example that is made of polyglycolic acid (hereinafter, the nonwoven fabric is also referred to as a "PGA sheet") were subjected to a burst strength test using lungs isolated from a rat as an adherend.

Specifically, both lungs including the trachea were isolated from a rat, a defect having a depth of 2 mm was produced in the pleura of the isolated lungs with a 20 G injection needle, and a sheet piece having a diameter of about 7 mm and a thickness of 0.2 mm was attached to cover the defect. At that time, hydration and gelation of the gelatin derivative fiber sheet piece were observed.

Then, the isolated lungs to which the sheet piece was attached were left standing for 10 minutes, and air was sent from the trachea at a flow rate of 1.0 mL/sec in a state in which the lungs were immersed in physiological saline at 37°C. The pressure when the sheet piece attached to the defect was broken and air leakage occurred due to expansion of the lungs was measured by a pressure gauge.

The results are shown in Fig. 6.

Fig. 6 is a graph showing the results of the burst strength test on the lungs isolated from the rat about the gelatin derivative fiber sheet (13C10) and the gelatin fiber sheet (Org) that were prepared with thermal crosslinking for 5 hours, and the nonwoven fabric made of polyglycolic acid (that is, the PGA sheet).

As shown in Fig. 6, the burst strength in the case of using the gelatin fiber sheet (Org) and the nonwoven fabric made of polyglycolic acid (that is, the PGA sheet) was about 20 (cmH₂O) and about 15 (cmH₂O), respectively, whereas the burst strength in the case of using the gelatin derivative fiber sheet (13C10) was more than 40 (cmH₂O).

In this burst strength test, it is considered that the reason why about 2.0 times as high a burst strength as that of the gelatin fiber sheet (Org) and about 2.7 times as high a burst strength as that of the nonwoven fabric made of polyglycolic acid (that is, the PGA sheet) were obtained when the gelatin derivative fiber sheet (13C10) was used is due to the hydrophobic interaction between the extracellular matrix and cell components, which are constituent components of the pleural surface, and the decanoyl group in the gelatin derivative.

### [Toxicity Test on Cells]

The gelatin derivative fiber sheet (13C10) and the gelatin fiber sheet (Org) prepared with thermal crosslinking for 5 hours in this example were subjected to a toxicity test on L-929 cells.

More specifically, L-929 cells were cultured on each sheet for 24 hours, and then the number of cells was counted by a WST-8 assay. As for the morphology of the cells, actin staining was performed using DAPI (a nucleic acid fluorescence staining reagent that is impermeable to cell membranes), and then the immobilized cells were observed with a fluorescence microscope.

As a result, as for the gelatin derivative fiber sheet (13C10), it was recognized that 90% or more of the cultured L-929 cells were alive, and the survival rate on the sheet was at the same level as that on the gelatin fiber sheet (Org).

### [Biocompatibility/Degradability Test]

The gelatin derivative fiber sheet (13C10) and the gelatin fiber sheet (Org) prepared with thermal crosslinking for 5 hours in this example and the nonwoven fabric made of polyglycolic acid (that is, the PGA sheet) were subjected to a biocompatibility test and a degradability test by subcutaneous implantation in the back of rats.

More specifically, the center of the back of each rat was shaved and disinfected, then the skin was incised, a sheet having a diameter of about 7 mm and a thickness of 0.2 mm was implanted into the back, and then the skin was sutured.

The observation period was set to 3 days, 7 days, 14 days, and 21 days after surgery, and the implanted sheet and the surrounding tissue were collected in each observation period and subjected to optical microscope observation and tissue observation by hematoxylin-eosin staining.

As a result, in the rat implanted with the gelatin derivative fiber sheet (13C10), the sheet was completely degraded on day 21 after surgery, and no strong inflammatory reaction was observed. More specifically, on day 14 after surgery, healing of the incision site and degradation of the sheet proceeded to such an extent that the incision site and the sheet could hardly be observed with the naked eye. From these results, it is considered that the degradation reaction of the gelatin derivative fiber sheet proceeded by the enzyme secreted from the tissue cells around the sheet as the skin incised at the time of sheet implantation healed.

On the other hand, in the rat implanted with the gelatin fiber sheet (Org), the degradation speed of the sheet was slightly slower than in the case of using the gelatin derivative fiber sheet (13C10), and the sheet was almost completely degraded on day 21 after surgery.

In contrast, in the rat implanted with the nonwoven fabric made of polyglycolic acid (that is, the PGA sheet), the sheet remained even on day 21 after surgery. This is considered to be because the degradation of the PGA sheet is a hydrolysis reaction, and thus the degradation speed of the sheet was slower than the progress of the healing of the incision site. In such a case, there is a concern about the possibility of continuation of an inflammatory reaction due to the sheet remaining after tissue healing.

### <Example 5>

A gelatin derivative (8C10-ApGltn) having a derivatization rate of 8 mol% was prepared by the same procedure as in Example 1 except that the amount of decanal added to the raw material gelatin solution was changed.

Using the gelatin derivative (8C10), a nonwoven fabric made of gelatin derivative fibers was prepared by the same procedure as in Example 1, and then the obtained nonwoven fabric was heated (thermally crosslinked) in a reduced pressure environment at 150°C for 5 hours to produce a gelatin derivative fiber sheet. Hereinafter, the gelatin derivative fiber sheet obtained in this example is also referred to as "8C10-AdFS".

### <Example 6>

In a UV irradiation box (produced by the National Institute for Materials Science), 8C10-AdFS obtained in Example 5 was left standing and irradiated with ultraviolet rays (source: a UV lamp, manufactured by MIYATA ELEVAM Inc.) of 185 nm and 254 nm at normal temperature for 60 minutes to surface-treat the fibers in the fiber sheet. Hereinafter, the gelatin derivative fiber sheet obtained in this example, which was subjected to UV irradiation for 60 minutes, is also referred to as "UV-8C10-AdFS".

### <Reference Example 2>

A gelatin fiber sheet (hereinafter also referred to as "Org-AdFS") obtained by subjecting the raw material gelatin (Org) of Reference Example 1 to thermal crosslinking for 5 hours was irradiated with ultraviolet rays by the same procedure as in Example 6 to surface-treat the fibers in the fiber sheet. Hereinafter, the gelatin fiber sheet obtained in this reference example, which was subjected to UV irradiation for 60 minutes, is also referred to as "UV-Org-AdFS".

As for the gelatin derivative (8C10-ApGltn) prepared in Example 5 and the raw material gelatin (Org), an absorption spectrum was measured by Fourier transform infrared spectroscopy (FT-IR), and the molecular structure was analyzed. As a result, in both cases, an absorption around 2925 to 2970 cm⁻¹ derived from -CH₂- stretching vibration and an absorption around 2875 cm⁻¹ derived from -CHs- stretching vibration of an alkyl terminal were observed. Meanwhile, in 8C10-ApGltn, the transmittance (%T) around 3280 cm⁻¹ derived from -NH- stretching vibration tended to be lower than that of Org.

As a result of measuring the ¹H-NMR spectrum, in 8C10-ApGltn, methylene protons derived from an alkylene group were observed around 1.27 ppm.

As a result of observation with a scanning electron microscope (SEM), it was recognized that 8C10-AdFS, UV-8C10-AdFS, Org-AdFS, and UV-Org-AdFS all had a fiber diameter in the range of 1 to 2 µm, and that fiber sheets having a uniform structure were obtained.

### [Evaluation of Physical Properties]

On the surfaces of 8C10-AdFS, UV-8C10-AdFS, Org-AdFS, and UV-Org-AdFS, 1 µL of pure water was dropped, and the contact angle of the water droplet after 1 second was measured (n = 3).

As a result, in 8C10-AdFS and UV-8C10-AdFS, the contact angle of the water droplet was about 85° and about 5°, respectively. As for gelatin derivative fiber sheets produced by setting the ultraviolet irradiation time for 8C10-AdFS to 10 minutes or 30 minutes, the measurement results of the contact angle were about 70° and about 10°, respectively. From these results, it was recognized that in the fiber sheet containing the gelatin derivative (8C10), the contact angle of the water droplet is significantly reduced as compared with the case of no irradiation, and the surfaces of the gelatin derivative fibers are hydrophilized by ultraviolet irradiation for 10 minutes, the surfaces of the gelatin derivative fibers become highly hydrophilic by irradiation for 30 minutes, and in addition, the surfaces of the gelatin derivative fibers become almost completely hydrophilic by irradiation for 60 minutes.

On the other hand, in Org-AdFS and UV-Org-AdFS, the contact angle of the water droplet was about 40° and about 0°, respectively. As for gelatin fiber sheets produced by setting the ultraviolet irradiation time for Org-AdFS to 5 minutes, 10 minutes, or 30 minutes, the measurement results of the contact angle were about 15°, about 7.5°, and about 0°, respectively. From these results, it was recognized that in the fiber sheet containing the raw material gelatin, the contact angle of the water droplet is greatly reduced as compared with the case of no irradiation, and the surfaces of the gelatin fibers become highly hydrophilic by ultraviolet irradiation for 10 minutes or less, and further, the surfaces of the gelatin fibers become almost completely hydrophilic by irradiation for 30 minutes.

A test piece was cut out from each of 8C10-AdFS, UV-8C10-AdFS, Org-AdFS, and UV-Org-AdFS, and the test piece was immersed in physiological saline at 37°C for 24 hours to evaluate the degree of swelling. As a result, all the sheets showed about the same swelling ratio in the range of about 12.5% to about 16%, and among them, UV-8C10-AdFS tended to have a slightly higher swelling ratio.

In addition, the fiber sheets molded into a dumbbell shape in accordance with JIS K 7127: 1999 were subjected to a tensile test. As a result, almost the same stress-strain curves were obtained for Org-AdFS and UV-Org-AdFS. On the other hand, the Young's modulus of 8C10-AdFS was lower than those of Org-AdFS and UV-Org-AdFS, and the Young's modulus of UV-8C10-AdFS was still lower than that of 8C10-AdFS.

These physical properties are considered to reflect the crosslinking density in the gelatin derivative fiber sheet described above or the structural change of the gelatin due to heat treatment. Therefore, it is presumed that these physical properties tend to vary depending on the derivatization rate in the gelatin, the thickness of the gelatin derivative fiber sheet, the thermal crosslinking conditions (thermal crosslinking time), and the like.

### [Burst Strength Test on Porcine Pleura]

Gelatin derivative fiber sheets produced by setting the ultraviolet irradiation time for 8C10-AdFS, UV-8C10-AdFS, and 8C10-AdFS to 30 minutes or 120 minutes were subjected to the burst strength test on a porcine pleura.

The results are shown in Fig. 7.

Fig. 7 is a graph showing a relation between the ultraviolet irradiation time (min) and the burst strength (cmH₂O) of the gelatin derivative fiber sheets.

As shown in Fig. 7, the gelatin derivative fiber sheets irradiated with ultraviolet rays under predetermined conditions exhibited superior burst strength to that of the fiber sheet (8C10-AdFS) not irradiated with ultraviolet rays (n = 3). In particular, the fiber sheet (UV-8C10-AdFS) prepared with ultraviolet irradiation for 60 minutes exhibited about 3 times or more the burst strength than that of 8C10-AdFS.

In addition, fiber sheets prepared by the same procedure as in Example 5, Example 6, or Reference Example 2 except that the thermal crosslinking time was 1 hour were subjected to the burst strength test on a porcine pleura with the standing time after attaching the sheets to the pleural tissue set to 0.5 minutes, 1 minute, 2 minutes, 5 minutes, or 10 minutes.

The results are shown in Fig. 8.

Fig. 8 is a graph showing a relation between the standing time (min) and the burst strength (cmH₂O) of a gelatin derivative fiber sheet (UV-8C10-AdFS1) and a gelatin fiber sheet (UV-Org-AdFS1) that were prepared with thermal crosslinking for 1 hour and ultraviolet irradiation for 60 minutes, and a gelatin derivative fiber sheet (8C10-AdFS1) and a gelatin fiber sheet (Org-AdFS1) that were not irradiated with ultraviolet rays in the burst strength test on a porcine pleura.

As shown in Fig. 8, in the case of UV-8C10-AdFS1 (open circles), the burst strength reached the maximum value in a standing time shorter than 10 minutes (more specifically, in about 2 minutes to 5 minutes), whereas in the case of 8C10-AdFS1 (open squares) not irradiated with ultraviolet rays, the burst strength was the highest when the standing time was 10 minutes.

Similarly, in the case of UV-Org-AdFS1 (filled triangles), the burst strength reached the maximum value in a standing time shorter than 10 minutes (more specifically, in about 5 minutes), whereas in the case of Org-AdFS1 (filled circles) not irradiated with ultraviolet rays, the burst strength was the highest when the standing time was 10 minutes.

These results suggest that the hydrophilization of the surfaces of the gelatin derivative fibers and the gelatin fiber sheets by ultraviolet irradiation increased the moisture absorption speed of the gelatin derivative fiber sheets from the pleural tissue, resulting in an improvement in the speed of adhesion between the gelatin fiber sheets and the pleural tissue. It was also found that the effect of improving the adhesion speed was more remarkable in the gelatin derivative fiber sheets than in the gelatin fiber sheets.

In the same manner as described above with respect to the burst strength test on the porcine pleura using the gelatin derivative fiber sheets of Examples 1 to 3, also in the case of the gelatin derivative fiber sheets produced using the gelatin derivative (8C10), it was possible to measure the pressure when the sheet was broken without separation at the adhesion site between the pleural tissue and the sheet while flowing physiological saline in the test chamber.

### [Degradability Test Using Collagenase]

UV-8C10-AdFS and UV-Org-AdFS were subjected to an enzymatic degradability test using a solution obtained by dissolving a collagenase in physiological saline (that is, a collagenase solution).

Specifically, a sample (UV-8C10-AdFS or UV-Org-AdFS) was placed in a container containing the collagenase solution, and immersed in the collagenase solution for 30 minutes, 60 minutes, 90 minutes, 120 minutes, 150 minutes, and 180 minutes. Then, the collagenase solution was removed, and the weight of the remaining sample was measured to calculate the residual rate (%) with respect to the weight of the sample before being immersed in the collagenase solution.

The results are shown in Fig. 9.

Fig. 9 is a graph showing results of the degradability test using the collagenase on UV-8C10-AdFS and UV-Org-AdFS.

As shown in Fig. 9, in UV-Org-AdFS (filled circles), more than 80% of the initial sheet weight remained after 90 minutes from the start of immersion in the collagenase solution, whereas in UV-8C10-AdFS (open circles), a decrease of more than 50% from the initial sheet weight was observed after 90 minutes from the start of immersion in the collagenase solution, the weight reduced to less than 10% after 120 minutes, and the sample was almost completely degraded after 150 minutes (n = 3).

### [Toxicity Test on Cells]

UV-8C10-AdFS and UV-Org-AdFS were subjected to a toxicity test on L-929 cells.

More specifically, L-929 cells were cultured on each sheet for 24 hours, and then the number of cells was counted by a WST-8 assay. As for the morphology of the cells, actin staining was performed using DAPI (a nucleic acid fluorescence staining reagent that is impermeable to cell membranes), and then the immobilized cells were observed with a fluorescence microscope.

As a control, the number of cells was counted by the WST-8 assay using cells obtained by culturing L-929 cells in a normal culture medium for 24 hours.

As a result, it was recognized that 90% or more of the cultured L-929 cells were alive for both UV-8C10-AdFS and UV-Org-AdFS.

In particular, in the case of UV-8C10-AdFS, the survival rate of the cultured L-929 cells was almost 100%, and in addition, the proliferation rate after 24 hours of culture was about 400%, which was significantly higher than those of UV-Org-AdFS (about 300%) and the control (about 220%).

### Industrial Applicability

Since the biological tissue adhesive sheet of the present invention has tissue adhesiveness superior to that of conventional medical sheets and also has excellent film strength, the sheet is expected to be applied to medical fields such as respiratory surgery, gastroenterological surgery, and gastroenterological medicine.

In addition, when the biological tissue adhesive sheet of the present invention contains an agent (for example, an anticancer agent) fixed to the nonwoven fabric made of fibers containing a crosslinked gelatin derivative, the sheet can be used not only as a tissue reinforcement material but also as an agent eluting sheet.

## Claims

1. A biological tissue adhesive sheet comprising a nonwoven fabric made of fibers containing a crosslinked gelatin derivative, the gelatin derivative being represented by Formula 1 shown below:
Formula 1: Gltn-NH-L-CHR¹R²
wherein Gltn represents a gelatin residue, L represents a single bond or a divalent linking group, R¹ and R² are each independently a hydrocarbon group having 1 to 20 carbon atoms or a hydrogen atom, and at least one selected from the group consisting of R¹ and R² is the hydrocarbon group, and
wherein the biological tissue adhesive sheet has a thickness in a range of 100 µm or more and 500 µm or less.

2. The biological tissue adhesive sheet as claimed in claim 1, wherein the hydrocarbon group is at least one selected from the group consisting of a chain hydrocarbon group having 1 to 20 carbon atoms, an alicyclic hydrocarbon group having 3 to 20 carbon atoms, an aromatic hydrocarbon group having 6 to 14 carbon atoms, and a group that is a combination of the foregoing groups and has 2 to 20 carbon atoms.

3. The biological tissue adhesive sheet as claimed in claim 1 or 2, wherein the hydrocarbon group is an alkyl group having 1 to 20 carbon atoms.

4. The biological tissue adhesive sheet as claimed in claim 3, wherein the alkyl group has 4 to 18 carbon atoms.

5. The biological tissue adhesive sheet as claimed in any one of claims 1 to 4, comprising an anticancer agent fixed to the nonwoven fabric.

6. The biological tissue adhesive sheet as claimed in any one of claims 1 to 4, comprising an antimicrobial agent fixed to the nonwoven fabric.

7. The biological tissue adhesive sheet as claimed in any one of claims 1 to 4, comprising a growth factor fixed to the nonwoven fabric.

8. The biological tissue adhesive sheet as claimed in any one of claims 1 to 7, wherein the gelatin derivative is a derivative of a cold-water fish gelatin.

9. The biological tissue adhesive sheet as claimed in claim 8, wherein the cold-water fish gelatin is at least one selected from the group consisting of cod gelatin, sea bream gelatin, salmon gelatin, and a genetically-engineered gelatin derived from cod, sea bream, or salmon.

10. A biological tissue reinforcement material kit comprising the biological tissue adhesive sheet as claimed in any one of claims 1 to 9, and an instruction describing how to use the biological tissue adhesive sheet and/or precautions for use.

11. A method for producing the biological tissue adhesive sheet as claimed in any one of claims 1 to 9, the method comprising:
preparing a composition containing the gelatin derivative and a solvent;
electrospinning the composition to prepare a nonwoven fabric made of fibers containing the gelatin derivative; and
applying energy to the nonwoven fabric to produce a biological tissue adhesive sheet having a thickness in a range of 100 µm or more and 500 µm or less.

12. The method as claimed in claim 11, wherein the energy is applied by heating the nonwoven fabric.

13. The method as claimed in claim 11 or 12, further comprising irradiating the biological tissue adhesive sheet with an ultraviolet ray to hydrophilize surfaces of the fibers containing the gelatin derivative.

14. The method as claimed in claim 13, wherein the biological tissue adhesive sheet is irradiated with the ultraviolet ray for 10 minutes to 90 minutes.

## Patentansprüche

1. Klebefolie für biologisches Gewebe, umfassend einen Vliesstoff, der aus Fasern gefertigt ist, welche ein vernetztes Gelatinederivat enthalten, wobei das Gelatinederivat durch die nachfolgend gezeigte Formel 1 dargestellt wird:
Formel 1: Gltn-NH-L-CHR¹R²
wobei Gltn einen Gelatinerest darstellt, L eine Einfachbindung oder eine zweiwertige Verbindungsgruppe darstellt, R¹ und R² jeweils unabhängig voneinander eine Kohlenwasserstoffgruppe sind, die 1 bis 20 Kohlenstoffatome oder ein Wasserstoffatom aufweist, und zumindest eines aus der Gruppe bestehend aus R1 und R2 die Kohlenwasserstoffgruppe ist, und
wobei die Klebefolie für biologisches Gewebe eine Dicke in einem Bereich von 100 µm oder mehr und 500 µm oder weniger aufweist.

2. Klebefolie für biologisches Gewebe nach Anspruch 1, wobei die Kohlenwasserstoffgruppe zumindest eine ist, welche aus der Gruppe ausgewählt ist, welche aus einer Ketten-Kohlenwasserstoffgruppe, die 1 bis 20 Kohlenstoffatome aufweist, einer alicyclischen Kohlenwasserstoffgruppe, die 3 bis 20 Kohlenstoffatome aufweist, einer aromatischen Kohlenwasserstoffgruppe, die 6 bis 14 Kohlenstoffatome aufweist und einer Gruppe besteht, welche eine Kombination der vorstehenden Gruppen ist und 2 bis 20 Kohlenstoffatome aufweist.

3. Klebefolie für biologisches Gewebe nach Anspruch 1 oder 2, wobei die Kohlenwasserstoffgruppe eine Alkylgruppe ist, die 1 bis 20 Kohlenstoffatome aufweist.

4. Klebefolie für biologisches Gewebe nach Anspruch 3, wobei die Alkylgruppe 4 bis 18 Kohlenstoffatome aufweist.

5. Klebefolie für biologisches Gewebe nach einem der Ansprüche 1 bis 4, welche ein an dem Vliesstoff fixiertes Antikrebsmittel umfasst.

6. Klebefolie für biologisches Gewebe nach einem der Ansprüche 1 bis 4, welche einen an dem Vliesstoff fixierten antimikrobiellen Wirkstoff umfasst.

7. Klebefolie für biologisches Gewebe nach einem der Ansprüche 1 bis 4, welche einen an dem Vliesstoff fixierten Wachstumsfaktor umfasst.

8. Klebefolie für biologisches Gewebe nach einem der Ansprüche 1 bis 7, wobei das Gelatinederivat ein Derivat einer Kaltwasserfischgelatine ist.

9. Klebefolie für biologisches Gewebe nach Anspruch 8, wobei die Kaltwasserfischgelatine zumindest eine ist, welche aus der Gruppe ausgewählt ist, welche aus Kabeljau-Gelatine, Goldbrasse-Gelatine, Lachs-Gelatine und einer gentechnisch veränderten Gelatine aus Kabeljau, Goldbrasse oder Lachs besteht.

10. Kit mit Verstärkungsmaterial für biologisches Gewebe, welches die Klebefolie für biologisches Gewebe nach einem der Ansprüche 1 bis 9 und eine Anweisung, die beschreibt, wie die Klebefolie für biologisches Gewebe zu verwenden ist und/oder Vorsichtsmaßnahmen für die Verwendung umfasst.

11. Verfahren zum Herstellen der Klebefolie für biologisches Gewebe nach einem der Ansprüche 1 bis 9, wobei das Verfahren umfasst:
Erzeugen einer Zusammensetzung, welche das Gelatinederivat und ein Lösungsmittel enthält; Elektrospinnen der Zusammensetzung, um einen Vliesstoff zu erzeugen, der aus Fasern gefertigt ist, welche das Gelatinederivat enthalten; und
Anwenden von Energie auf den Vliesstoff, um eine Klebefolie für biologisches Gewebe herzustellen, die eine Dicke in einem Bereich von 100 µm oder mehr und 500 µm oder weniger aufweist.

12. Verfahren nach Anspruch 11, wobei die Energie durch Erhitzen des Vliesstoffes angewendet wird.

13. Verfahren nach Anspruch 11 oder 12, weiter Bestrahlen der Klebefolie für biologisches Gewebe mit einer Ultraviolettstrahlung umfassend, um die Oberflächen der Fasern, die das Gelatinederivat enthalten, zu hydrophilieren.

14. Verfahren nach Anspruch 13, wobei die Klebefolie für biologisches Gewebe 10 Minuten bis 90 Minuten lang mit der Ultraviolettstrahlung bestrahlt wird.

## Revendications

1. Feuille adhésive de tissu biologique comprenant un tissu non tissé composé de fibres contenant un dérivé de gélatine réticulé, le dérivé de gélatine étant représenté par la formule 1 ci-dessous :
Formule 1: Gltn-NH-L-CHR¹R²
dans laquelle Gltn représente un résidu de gélatine, L représente une liaison simple ou un groupe de liaison divalent, R¹ et R² sont chacun indépendamment un groupe hydrocarboné présentant 1 à 20 atomes de carbone ou un atome d'hydrogène, et au moins un sélectionné dans le groupe composé de R1 et R2 est le groupe hydrocarboné, et
dans laquelle la feuille adhésive de tissu biologique présente une épaisseur dans une plage allant de 100 µm ou plus à 500 µm ou moins.

2. Feuille adhésive de tissu biologique selon la revendication 1, dans laquelle le groupe hydrocarboné est au moins un groupe choisi dans le groupe composé d'un groupe hydrocarboné en chaîne présentant 1 à 20 atomes de carbone, d'un groupe hydrocarboné alicyclique présentant 3 à 20 atomes de carbone, d'un groupe hydrocarboné aromatique présentant 6 à 14 atomes de carbone, et d'un groupe qui est une combinaison des précédents groupes et présente 2 à 20 atomes de carbone.

3. Feuille adhésive de tissu biologique selon la revendication 1 ou 2, dans laquelle le groupe hydrocarboné est un groupe alkyle présentant 1 à 20 atomes de carbone.

4. Feuille adhésive de tissu biologique selon la revendication 3, dans laquelle le groupe alkyle présente 4 à 18 atomes de carbone.

5. Feuille adhésive de tissu biologique selon l'une quelconque des revendications 1 à 4, comprenant un agent anticancéreux fixé au tissu non tissé.

6. Feuille adhésive de tissu biologique selon l'une quelconque des revendications 1 à 4, comprenant un agent antimicrobien fixé au tissu non tissé.

7. Feuille adhésive de tissu biologique selon l'une quelconque des revendications 1 à 4, comprenant un facteur de croissance fixé au tissu non tissé.

8. Feuille adhésive de tissu biologique selon l'une quelconque des revendications 1 à 7, dans laquelle le dérivé de gélatine est un dérivé d'une gélatine de poisson d'eau froide.

9. Feuille adhésive de tissu biologique selon la revendication 8, dans laquelle la gélatine de poisson d'eau froide est au moins une gélatine sélectionné dans le groupe composée de la gélatine de morue, de la gélatine de daurade, de la gélatine de saumon et d'une gélatine génétiquement modifiée dérivée de morue, de daurade, ou de saumon.

10. Kit de matériau de renforcement de tissu biologique comprenant la feuille adhésive de tissu biologique selon l'une quelconque des revendications 1 à 9, et une instruction décrivant de quelle manière utiliser la feuille adhésive de tissu biologique et/ou des précautions d'emploi.

11. Procédé de production de la feuille adhésive de tissu biologique selon l'une quelconque des revendications 1 à 9, le procédé comprenant :
la préparation d'une composition contenant le dérivé de gélatine et un solvant ; l'électrofilage de la composition pour préparer un tissu non tissé composé de fibres contenant le dérivé de gélatine ; et
l'application de l'énergie au tissu non tissé pour produire une feuille adhésive de tissu biologique présentant une épaisseur dans une plage allant de 100 µm ou plus à 500 µm ou moins.

12. Procédé selon la revendication 11, dans lequel l'énergie est appliquée en chauffant le tissu non tissé.

13. Procédé selon la revendication 11 ou 12, comprenant en outre l'irradiation de la feuille adhésive de tissu biologique avec un rayonnement ultraviolet pour hydrophiliser des surfaces des fibres contenant le dérivé de gélatine.

14. Procédé selon la revendication 13, dans lequel la feuille adhésive de tissu biologique est irradiée avec le rayonnement ultraviolet pendant 10 minutes à 90 minutes.
